# EUROPÄISCHE PATENTSCHRIFT

(11) **EP 3 714 822 B1**
(45) Veröffentlichungstag und Bekanntmachung des Hinweises auf die Patenterteilung: **18.01.2023**
(21) Anmeldenummer: 19164955.7
(22) Anmeldetag: 25.03.2019
(51) Int. Cl.: A61B 18/04, G01F 1/34, G01F 7/00, G01F 1/44, G01F 1/36, G01F 15/00, G05D 7/06, A61B 18/00, G01F 1/32

(54) **FLUIDSTEUERANORDNUNG FÜR EIN MEDIZINISCHES GERÄT**
FLUID CONTROL ASSEMBLY FOR A MEDICAL DEVICE
DISPOSITIF DE COMMANDE DE FLUIDE POUR UN APPAREIL MÉDICAL

(43) Veröffentlichungstag der Anmeldung: 30.09.2020
(60) Teilanmeldung: 22208817.1
(73) Patentinhaber: Erbe Elektromedizin GmbH, 72072 Tübingen (DE)
(72) Erfinder: DAWIDOWSKY, Uwe, 72108 Rottenburg (DE); HAISCH, Philipp, 72076 Tübingen (DE)
(74) Vertreter: Rüger Abel Patentanwälte PartGmbB

(56) Entgegenhaltungen:
- DE-A1- 10 127 261
- US-A1- 2005 011 282
- US-B2- 9 374 891

## Beschreibung

Die Erfindung betrifft eine Fluidsteueranordnung für ein medizinisches Gerät. Mittels der Fluidsteueranordnung kann der Volumenstrom oder Massenstrom eines Fluids, insbesondere eines Gases für medizinische Anwendungen, gesteuert oder geregelt werden.

Vorrichtungen zur Fluidsteuerung sind aus verschiedenen technischen Gebieten bekannt. US 5,888,390 A beschreibt eine Miniatur-Anordnung aus faltbar verbundenen metallischen Platten, in deren jeweilige Innenseiten Fluidkanalaussparungen eingeätzt werden. Durch Zusammenfalten entsteht ein mehrlagiger Körper, um Fluide zu leiten. Diese Anordnung ist aufgrund der Miniaturisierung für Analyseinstrumente, beispielsweise Chromatographen geeignet.

DE 195 46 535 A1 offenbart ein Verfahren und eine Vorrichtung zur Probenentnahme mit integrierter analytischchemischer Sensor-Messung und ein Verfahren zur Herstellung der Vorrichtung. Eine Messkartusche ist mit Chemo- oder Biosensoren bestückt und kann über Luer-Steckverbindungen zwischen Nadel und Spritze angeordnet werden. Nach der Probenentnahme mittels der Spritze kann die Messkartusche in ein Handmessgerät eingelegt werden.

Aus US 5,020,373 ist ein Strömungsmessgerät bekannt, das einen Strömungssensor aufweist, der in einem Kanalabschnitt eines Strömungskanals mit reduziertem Durchmesser angeordnet ist. In Strömungsrichtung stromabwärts ist ein Wirbelerzeuger sowie ein Druckmesskanal angeordnet. Die erzeugten Wirbelfrequenzen sind eine Funktion der Strömungsrate, die über den Druckmesskanal erfasst werden kann.

WO 99/36747 A1 offenbart eine Vorrichtung und ein Verfahren zur Erfassung der Fluidströmung als eine Funktion der Druckdifferenz zwischen zwei Anschlüssen in einem Strömungskanal.

US 2005/0011282 A1 beschreibt eine Einrichtung zum optischen Anzeigen einer Fluidströmung. Ein Schaltkreis mit einem Strömungssensor steuert abhängig von einer Gasströmung durch ein Gehäuse eine LED. Das Gehäuse besteht aus zwei Gehäuseteilen, die den Schaltkreis aufnehmen. Der Schaltkreis ist auf einer Leiterplatte angeordnet. Durch das eine Gehäuseteil kann Gas einströmen und durch das jeweils andere Gehäuseteil ausströmen.

US 9,374,891 B2 beschreibt einen Träger für einen Schaltkreis aufweisend eine Leiterplatte.

Aus DE 101 27 261 A1 ist eine Messvorrichtung für die Strömungsrate eines Gases bekannt, die zwei Strömungssensoren aufweist. Die Strömungssensoren sind in einem Strömungsraum angeordnet und haben unterschiedliche Messkennlinien.

Ausgehend von dem Stand der Technik kann es als Aufgabe der vorliegenden Erfindung angesehen werden, eine verbesserte Fluidsteueranordnung für ein medizinisches Gerät zu schaffen. Insbesondere soll die Fluidsteueranordnung dazu eingerichtet sein, eine Gasströmung eines medizinischen Gases zu steuern und dabei einen kompakten und kostengünstigen Aufbau zu ermöglichen.

Diese Aufgabe wird mit der Fluidsteueranordnung mit den Merkmalen des Patentanspruches 1 gelöst.

Die Fluidsteueranordnung ist insbesondere dazu eingerichtet, eine Gasströmung eines Gases, wie etwa Argon, Sauerstoff, Kohlendioxid oder ein anderes in einem medizinischen Gerät verwendetes Gas zu steuern oder zu regeln. Anstelle einer Gasströmung kann bei anderen medizinischen Anwendungen auch eine Flüssigkeitsströmung gesteuert oder geregelt werden. Bei dem medizinischen Gerät kann es sich insbesondere um ein Gerät zur Argonplasma-Koagulation handeln.

Die Fluidsteueranordnung hat einen Fluidsteuerkreis, der eine Fluidkanalanordnung und wenigstens ein Fluidsteuerbauteil hat. Die Fluidkanalanordnung und das Fluidsteuerbauteil sind zwischen einem Eingangsanschluss und einem Ausgangsanschluss angeordnet und bilden gemeinsam eine fluidische Verbindung zwischen dem Eingangsanschluss und dem Ausgangsanschluss. Vorzugsweise ist wenigstens ein Fluidsteuerbauteil oder sind sämtliche vorhandene Fluidsteuerbauteile beim Betrieb der Fluidsteueranordnung fluiddurchströmt. Bei einem Fluidsteuerbauteil kann es sich beispielsweise um ein Wegeventil, ein Proportionalventil, ein Drucksteuerventil oder Druckregelventil, einen Filter oder ein anderes fluiddurchströmbares Bauteil handeln, das nicht nur ein Fluid leitet, sondern zusätzlich eine Fluideigenschaft (z.B. Druck, Volumenstrom, Massenstrom, Reinheit) des durchgeleiteten Fluids beeinflusst.

Ein Steuerschaltkreis ist dazu eingerichtet, das wenigstens eine Fluidsteuerbauteil des Fluidsteuerkreises zu steuern. Hierfür weist der Steuerschaltkreis wenigstens ein elektrisches und/oder elektronisches Bauteil auf, umfassend beispielsweise eine Steuereinheit, das elektrische Steuersignale für das wenigstens eine Fluidsteuerbauteil bereitstellt. Vorzugsweise weist der Steuerschaltkreis keine Bauteile auf, die fluiddurchströmt sind. Ein oder mehrere Bauteile des Steuerschaltkreises, beispielsweise Drucksensoren, können in Kontakt mit dem Fluid sein, sind aber vorzugsweise nicht fluiddurchströmt.

Die Fluidsteueranordnung weist ein erstes Trägerteil mit einer ersten Montagefläche und einer ersten Kopplungsfläche sowie ein zweites Trägerteil mit einer zweiten Montagefläche und einer zweiten Kopplungsfläche auf. Die Montagefläche und die Kopplungsfläche eines jeweiligen Trägerteils sind vorzugsweise auf entgegengesetzten Seiten des betreffenden Trägerteils angeordnet. Die erste Montagefläche und/oder die zweite Montagefläche erstrecken sich bei einem Ausführungsbeispiel jeweils in einer Ebene. Die erste Montagefläche ist für das Anbringen wenigstens eines Fluidsteuerbauteils eingerichtet. Die zweite Montagefläche ist für das Anbringen wenigstens eines elektrischen und/oder elektronischen Bauteils des Steuerschaltkreises eingerichtet Ein Fluidsteuerbauteil oder mehrere Fluidsteuerbauteile des Fluidsteuerkreises sind an der ersten Montagefläche des ersten Trägerteils und/oder ein Bauteil oder mehrere Bauteile des Steuerschaltkreises sind an der zweiten Montagefläche des zweiten Trägerteils angeordnet. Es ist bevorzugt, wenn sämtliche Fluidsteuerbauteile des Fluidsteuerkreises und/oder sämtliche Bauteile des Steuerschaltkreises mittelbar oder unmittelbar an der jeweiligen Montagefläche angeordnet sind.

Bei einer Ausführungsform sind einige oder mehrere der vorhandenen Fluidsteuerbauteile unmittelbar an der ersten Montagefläche angeordnet und fluidisch mit der Fluidkanalanordnung verbunden. An der zweiten Montagefläche kann eine Leiterplattenanordnung befestigt sein, die wenigstens ein elektrisches und/oder elektronisches Bauteil des Steuerschaltkreises trägt. Bevorzugt sind sämtliche elektrischen und/oder elektronischen Bauteile des Steuerschaltkreises an der Leiterplattenanordnung angeordnet.

Es ist außerdem vorteilhaft, wenn an dem ersten Trägerteil und/oder an dem zweiten Trägerteil außerhalb der jeweiligen Montagefläche keine Bauteile befestigt sind.

Zur Bildung wenigstens eines Hauptfluidkanals der Fluidkanalanordnung ist in der ersten Kopplungsfläche wenigstens eine erste Fluidkanalaussparung und/oder in der zweiten Kopplungsfläche wenigstens eine zweite Fluidkanalaussparung vorhanden. Im Bereich der ersten Kopplungsfläche und der zweiten Kopplungsfläche sind das erste Trägerteil und das zweite Trägerteil miteinander verbunden. Die Kopplungsflächen können jeweils einen - vorzugsweise ebenen - Flächenabschnitt aufweisen, die flächig unmittelbar aneinander anliegen oder die unter Bildung eines Zwischenraums einander zugewandt sein. Diese Flächenabschnitte umgeben die Fluidkanalaussparungen der Trägerteile. Die wenigstens eine erste und zweite Fluidkanalaussparung ist gegenüber dem umgebenden Flächenabschnitt der ersten bzw. zweiten Kopplungsfläche vertieft ausgebildet. Wenn die beiden Trägerteile im Bereich ihrer Kopplungsflächen miteinander verbunden sind, begrenzt die erste Fluidkanalaussparung gemeinsam mit dem zweiten Trägerteil und/oder die zweite Fluidkanalaussparung gemeinsam mit dem ersten Trägerteil einen Hauptfluidkanal im Bereich der Trennstelle zwischen den beiden Trägerteilen. Die Trägerteile werden im Bereich der Kopplungsflächen derart miteinander verbunden, dass der wenigstens eine Hauptfluidkanal im Bereich der Trennstelle zwischen den beiden Trägerteilen fluidisch abgedichtet ist, unter Verwendung einer Dichtungsanordnung.

Der wenigstens eine Hauptfluidkanal ist ausschließlich im Bereich der Trennstelle bzw. Trennebene zwischen den Trägerteilen vorhanden und durch beide Trägerteile begrenzt. Weitere Fluidkanäle der Fluidkanalanordnung können innerhalb des ersten Trägerteils und/oder des zweiten Trägerteils verlaufen und insbesondere Abzweigkanäle von oder zu einer Fluidkanalaussparung bilden. Vorzugsweise sind sämtliche Fluidkanäle der Fluidkanalanordnung durch Kanalwände begrenzt, die entweder integraler Bestandteil des ersten Trägerteils oder integraler Bestandteil des zweiten Trägerteils sind. Innerhalb des Fluidsteuerkreises zwischen dem Eingangsanschluss und dem Ausgangsanschluss ist vorzugsweise keine fluidische Verbindung durch eine separate Leitung gebildet, die vollständig außerhalb der Trägerteile verläuft, beispielsweise unmittelbar zwischen zwei Fluidsteuerbauteilen.

Es ist bevorzugt, wenn jeder Hauptfluidkanal durch jeweils eine erste Fluidkanalaussparung im ersten Trägerteil und eine zweite Fluidkanalaussparung im zweiten Trägerteil gebildet ist. Jede erste Fluidkanalaussparung und/oder zweite Fluidkanalaussparung hat bevorzugt einen halbkreisförmigen Querschnitt. Somit kann bei hergestellter Verbindung zwischen den Trägerteilen jeder Hauptfluidkanal einen kreisförmigen Querschnitt aufweisen. Zumindest einige der Hauptfluidkanäle haben vorzugsweise einen im Wesentlichen kreisförmigen Querschnitt.

Die Fluidkanalanordnung kann wenigstens einen von einem Hauptfluidkanal abzweigenden fluidischen Abzweigkanal aufweisen. Beispielsweise kann im ersten Trägerteil wenigstens ein fluidischer erster Abzweigkanal vorhanden sein, der sich zwischen der ersten Montagefläche und der ersten Kopplungsfläche erstreckt. Entsprechend kann im zweiten Trägerteil wenigstens ein fluidischer zweiter Abzweigkanal vorhanden sein, der sich zwischen der zweiten Montagefläche und der zweiten Kopplungsfläche erstreckt.

Zur Herstellung der Trägerteile ist es besonders vorteilhaft, wenn das erste Trägerteil und das zweite Trägerteil durch jeweils ein Spritzgussteil gebildet sind. Vorzugsweise sind die Spritzgussteile aus einem einheitlichen Material, insbesondere Kunststoff- oder Verbundwerkstoffmaterial hergestellt. Das erste Trägerteil und das zweite Trägerteil sind dadurch als integrale Trägerteile ohne Naht- und Fügestelle ausgebildet. Insbesondere sind nach dem Herstellen der Trägerteile keine materialabtragenden Nacharbeiten zur Bildung von Fluidkanälen erforderlich. Bevorzugt werden sämtliche Fluidkanäle der Fluidkanalanordnung beim und durch das Herstellen der Trägerteile gebildet.

Ist im ersten Trägerteil wenigstens ein erster Abzweigkanal vorhanden, ist der wenigstens eine erste Abzweigkanal zumindest in einer Erstreckungsrichtung von der ersten Montagefläche zur ersten Kopplungsfläche oder umgekehrt von der ersten Kopplungsfläche zur ersten Montagefläche hinterschneidungsfrei. Der wenigstens eine erste Abzweigkanal kann konisch oder zylindrisch sein. Ist im ersten Trägerteil wenigstens ein zweiter Abzweigkanal vorhanden, ist der wenigstens eine zweite Abzweigkanal zumindest in einer Erstreckungsrichtung von der zweiten Montagefläche zur zweiten Kopplungsfläche oder umgekehrt von der zweiten Kopplungsfläche zur zweiten Montagefläche hinterschneidungsfrei. Der wenigstens eine zweite Abzweigkanal kann konisch oder zylindrisch sein. Dadurch vereinfacht sich die Herstellung der Trägerteile als Spritzgussteile erheblich.

Bevorzugt sind sämtliche sich quer zu den Montageflächen und Kopplungsflächen erstreckenden Aussparungen, zumindest in einer Richtung von der betreffenden Kopplungsfläche zur betreffenden Montagefläche oder umgekehrt von der betreffenden Montagefläche zur betreffenden Kopplungsfläche hinterschneidungsfrei. Der wenigstens eine erste Abzweigkanal und/oder der wenigstens eine zweite Abzweigkanal erstrecken sich insbesondere in einer Formschließrichtung einer Spritzgussform zur Herstellung des ersten Trägerteils bzw. des zweiten Trägerteils als Spritzgussteil.

Die vorstehend erläuterte Herstellung der Trägerteile als Spritzgussteile und/oder die hinterschneidungsfreie Ausgestaltung des wenigstens einen Abzweigkanals ist ein unabhängiger Aspekt der Erfindung und kann insbesondere unabhängig davon vorgesehen werden, ob die Fluidsteuerbauteile des Fluidsteuerkreises und/oder die Bauteile des Steuerschaltkreises an den Montageflächen angeordnet sind.

Zwischen der ersten Kopplungsfläche und der zweiten Kopplungsfläche ist eine Dichtungsanordnung zur Abdichtung des wenigstens einen Hauptfluidkanals vorhanden. Die Dichtungsanordnung kann für jeden vorhandenen Hauptfluidkanal jeweils eine Ringdichtung aufweisen. Sind im Bereich der Trennstelle bzw. Trennebene zwischen den Trägerteilen mehrere separate Hauptfluidkanäle ausgebildet, ist jeder der Hauptfluidkanäle vorzugsweise von einer Ringdichtung der Dichtungsanordnung vollständig umschlossen, um den Hauptfluidkanal gegenüber der Umgebung abzudichten. Hierfür kann entweder in der ersten Kopplungsfläche und/oder in der zweiten Kopplungsfläche eine Ringnut zum Einlegen der Ringdichtung vorhanden sein. Die Ringnut kann eine erste Fluidkanalaussparung in der ersten Kopplungsfläche vollständig umschließen oder die Ringnut kann eine zweite Fluidkanalaussparung in der zweiten Kopplungsfläche vollständig umschließen.

Es ist außerdem vorteilhaft, wenn das erste Trägerteil und/oder das zweite Trägerteil zumindest teilweise aus einem transparenten Material hergestellt ist, um bei hergestellter Verbindung zwischen den Trägerteilen von außen durch eine Sichtkontrolle beurteilen zu können, ob die Dichtungsanordnung korrekt im Bereich der Trennstelle zwischen den beiden Trägeranordnungen angeordnet ist. Insbesondere kann beurteilt werden, ob die wenigstens eine Ringdichtung korrekt in der zugeordneten Ringnut angeordnet ist.

Bevorzugt sind alle fluiddurchströmten Fluidsteuerbauteile der Fluidsteuereinheit an der ersten Montagefläche angeordnet und insbesondere unmittelbar angeordnet. Zusätzlich oder alternativ sind alle elektrischen und/oder elektronischen Bauteile des Steuerschaltkreises mittelbar oder unmittelbar an der zweiten Montagefläche angeordnet, vorzugsweise mittelbar über einer Leiterplattenanordnung. Bevorzugt ist keines der Bauteile des Steuerschaltkreises fluiddurchströmt.

Es ist vorteilhaft, wenn ein Hauptfluidkanal der Fluidkanalanordnung einen Strömungsmesskanal bildet. Der Strömungsmesskanal ist insbesondere Bestandteil einer Strömungsmesseinrichtung der Fluidsteueranordnung. Der Strömungsmesskanal hat bevorzugt an jeder Stelle einen im Wesentlichen kreisrunden Querschnitt. Wenigstes ein Druckmesskanalpaar mit zwei Druckmesskanälen ist vorhanden, deren Druckmesskanäle in Strömungsrichtung mit Abstand zueinander in den Strömungsmesskanal einmünden. Die Druckmesskanäle eines Druckmesskanalpaars sind vorzugsweise in demselben Trägerteil vorhanden, können alternativ auch in unterschiedlichen Trägerteilen vorhanden sein. Bevorzugt verlaufen die Druckmesskanäle zumindest abschnittsweise im und durch das zweite Trägerteil. Jedem der Druckmesskanäle ist bei einer bevorzugten Ausführungsform ein separater Drucksensor zugeordnet, der dazu eingerichtet ist, den Druck im Druckmesskanal und somit an der Mündungsstelle zwischen dem betreffenden Druckmesskanal und dem Strömungsmesskanal zu messen. Alternativ dazu ist an die Druckmesskanäle eines Druckmesskanalpaares ein Differenzdrucksensor angeschlossen, so dass für jedes Druckmesskanalpaar ein einziger Differenzdrucksensor ausreichend ist. Der Differenzdrucksensor ist dazu eingerichtet ein Differenzdrucksignal zu erzeugen, das und an die Steuereinheit zu übermitteln.

Zumindest ein Abschnitt jedes Druckmesskanals kann durch einen vorstehend beschriebenen Abzweigkanal gebildet sein. Bei einer bevorzugten Ausführungsform kann ein weiterer Abschnitt jedes Druckmesskanals in einem sich über die Montagefläche hinaus erstreckenden Stutzen verlaufen, der insbesondere integral mit dem zugehörigen Trägerteil ausgebildet ist.

Es ist außerdem vorteilhaft, wenn ein erstes Druckmesskanalpaar sowie ein zweites Druckmesskanalpaar vorhanden ist, deren Druckmesskanäle in denselben Strömungsmesskanal einmünden. Der Strömungsmesskanal hat bevorzugt einen ersten Kanalabschnitt mit einem ersten Strömungsquerschnitt und einen zweiten Kanalabschnitt mit einem zweiten Strömungsquerschnitt, wobei die beiden Strömungsquerschnitte voneinander verschieden sind und insbesondere unterschiedlich groß sind. Die Druckmesskanäle des ersten Druckmesskanalpaars münden in den ersten Kanalabschnitt und die Druckmesskanäle des zweiten Druckmesskanalpaares münden in den zweiten Kanalabschnitt ein. Bei dieser Ausgestaltung besteht die Möglichkeit, eine Strömungsmessung mit jedem Druckmesskanalpaar zu realisieren, wobei durch die unterschiedlichen Strömungsquerschnitte unterschiedliche Messbereiche zur Bestimmung des Volumenstroms bzw. des Massenstroms der Fluidströmung möglich sind. Insgesamt ist der dadurch zur Verfügung stehende Gesamtmessbereich vergrößert.

Zur Bestimmung des Volumenstroms bzw. des Massenstroms des Fluids durch den Strömungsmesskanal ist vorzugsweise eine Auswerteeinheit des Steuerschaltkreises vorhanden, der die gemessenen Druckwerte zumindest eines Druckmesskanalpaares, das einem gemeinsamen Druckmesskanalpaar zugeordnet ist, übermittelt werden. Aufgrund der Rohrreibung im Strömungsmesskanal entsteht zwischen den Druckmesskanälen eines gemeinsamen Druckmesskanalpaares ein Differenzdruck, der charakteristisch ist für den Volumenstrom bzw. den Massenstrom des Fluids durch den Strömungsmesskanal, der somit in der Auswerteeinheit ermittelt werden kann.

Die vorstehend erläuterte Ausgestaltung der Strömungsmesseinrichtung, insbesondere des Strömungsmesskanals und/oder der Druckmesskanäle und/oder der angeschlossenen Drucksensoren, kann auch zusätzlich oder alternativ zu anderen Aspekten der Erfindung realisiert werden.

Es ist außerdem bevorzugt, wenn der Fluidsteuerkreis eine Reihenschaltung aus einem Stellglied, insbesondere einem Proportionalventil sowie einer Strömungsmesseinrichtung aufweist. Die Strömungsmesseinrichtung kann insbesondere wie vorstehend beschrieben ausgebildet sein. In Strömungsrichtung befindet sich die Strömungsmesseinrichtung vorzugsweise vor dem Stellglied. Dadurch ist eine Strömungsmessung unabhängig von der an den Ausgangsanschluss angeschlossenen Last, beispielsweise einem chirurgischen Instrument, sichergestellt.

Es ist weiterhin vorteilhaft, wenn sich die Strömungsmesseinrichtung in Strömungsrichtung zwischen einer Druckbegrenzungseinrichtung oder einer Drucksteuer- oder Druckregeleinrichtung und dem Stellglied befindet. Vorzugsweise sind zwischen der Druckbegrenzungseinrichtung oder der Drucksteuer- oder Druckregeleinrichtung und dem Stellglied keine zusätzlichen Fluidsteuerbauteile - insbesondere keine den Fluiddruck beeinflussenden Fluidsteuerbauteile - vorhanden, die nicht zur Strömungsmesseinrichtung gehören.

Der Schaltungsaufbau des Fluidsteuerkreises im Hinblick auf den Hydraulikschaltplan, insbesondere die Anordnung der Strömungsmesseinrichtung in Reihe zu weiteren Fluidsteuerbauteilen, kann unabhängig von oder zusätzlich zu den vorbeschriebenen Merkmalen der Fluidsteueranordnung verwendet werden und ist insbesondere unabhängig davon, ob die Bauteile des Fluidsteuerkreises und/oder des Steuerschaltkreises an den Montageflächen der Trägerteile angeordnet sind.

Vorteilhafte Ausgestaltungen der Erfindung ergeben sich aus den abhängigen Patentansprüchen und den Zeichnungen. Nachfolgend werden bevorzugte Ausführungsbeispiele der Erfindung anhand der beigefügten Zeichnungen im Einzelnen erläutert. Es zeigen:
Figur 1 ein Blockschaltbild eines Ausführungsbeispiels eines medizinischen Geräts in Form eines Argonplasma-Koagulationsgeräts,
Figur 2 ein hydraulisches Blockschaltbild eines Ausführungsbeispiels eines Fluidsteuerkreises für das Gerät aus Figur 1,
Figur 3 eine schematische Prinzipdarstellung eines Ausführungsbeispiels einer Fluidsteueranordnung aufweisend einen Fluidsteuerkreis und einen Steuerschaltkreis, die an Trägerteilen angeordnet sind,
Figur 4 eine schematische Darstellung eines Ausführungsbeispiels eines ersten Trägerteils in einer Draufsicht auf eine erste Kopplungsfläche des ersten Trägerteils sowie eine schematische Darstellung eines Ausführungsbeispiels einer Dichtungsanordnung,
Figur 5 das erste Trägerteil aus Figur 4 in einem Schnittbild gemäß Schnittlinie V-V in Figur 4,
Figur 6 eine schematische Darstellung eines Ausführungsbeispiels eines zweiten Trägerteils in einer Draufsicht auf eine zweite Kopplungsfläche des zweiten Trägerteils,
Figur 7 das zweite Trägerteil gemäß Figur 6 in einer schematischen Seitenansicht gemäß Pfeil VII in Figur 6,
Figur 8 eine schematische Schnittdarstellung durch die miteinander verbundenen Trägerteile der Fluidsteueranordnung gemäß Figur 3,
Figur 9 eine Prinzipdarstellung der Anordnung eines Drucksensors des Steuerschaltkreises und
Figur 10 eine Prinzipdarstellung eines Ausführungsbeispiels einer Strömungsmesseinrichtung der Fluidsteueranordnung.

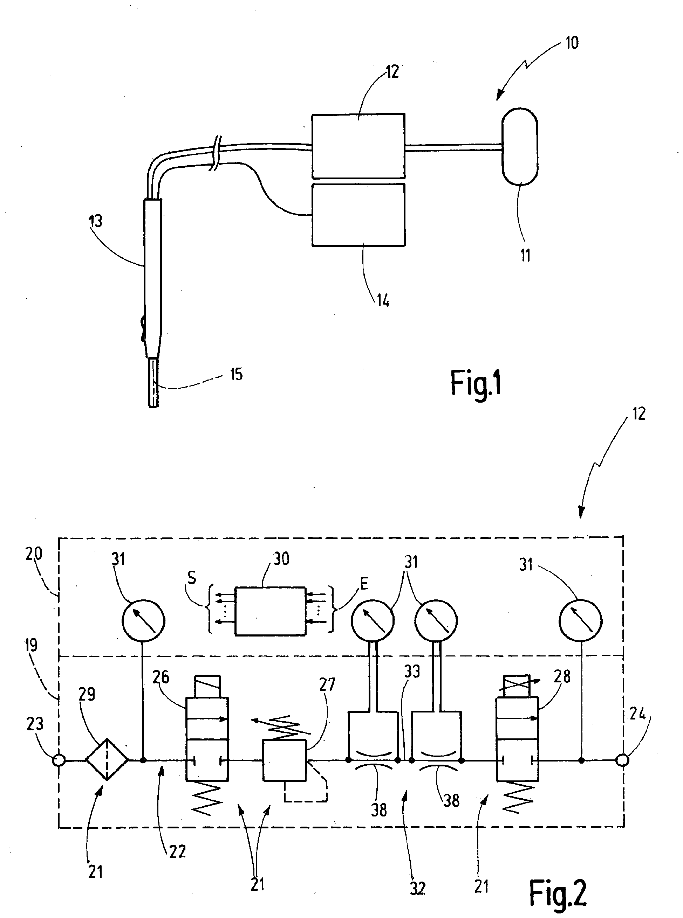

Figur 1 zeigt ein medizinisches Gerät 10, das beispielsgemäß als Argonplasma-Koagulationsgerät ausgebildet ist. Das medizinische Gerät 10 weist eine Fluidquelle 11 auf, an die eine Fluidsteueranordnung 12 fluidisch angeschlossen ist. An die Fluidsteueranordnung 12 ist ein Instrument anschließbar, durch das die Fluidströmung, die die Fluidsteueranordnung 12 bereitstellt, geleitet werden kann. Bei dem hier veranschaulichten Ausführungsbeispiel des medizinischen Geräts 10 ist außerdem eine Hochspannungsquelle 14 zum Bereitstellen einer Hochspannung für das Instrument 13 vorhanden. Das Instrument 13 kann elektrisch mit der Hochspannungsquelle 14 verbunden werden.

Durch das Instrument wird beim Ausführungsbeispiel durch die Fluidsteueranordnung 12 eine Argon-Gasströmung für das Instrument 13 bereitgestellt, die am Austrittsende des Instruments 13 um eine Elektrode 15 strömt. An die Elektrode 15 kann eine Hochspannung angelegt werden zur Argonplasma-Koagulation.

Das in Figur 1 veranschaulichte medizinische Gerät 10 ist lediglich ein Beispiel. Die erfindungsgemäße Fluidsteueranordnung 12 kann auch für andere medizinische Geräte 10 eingesetzt werden.

Die Fluidsteueranordnung 12 weist einen Fluidsteuerkreis 19 und einen Steuerschaltkreis 20 auf. Der Fluidsteuerkreis 19 umfasst wenigstens ein und beim Ausführungsbeispiel mehrere Fluidsteuerbauteile 21, die fluidisch an eine Fluidkanalanordnung 22 des Fluidsteuerkreises 19 angeschlossen sind. Die Fluidkanalanordnung 22 und die Fluidsteuerbauteile 21 stellen eine fluidische Verbindung zwischen einem Eingangsanschluss 23 und einem Ausgangsanschluss 24 der Fluidsteueranordnung 12 bzw. des Fluidsteuerkreises 19 her. An den Eingangsanschluss 23 kann die Fluidquelle 11 angeschlossen werden. An den Ausgangsanschluss 24 kann das Instrument 13 fluidisch angeschlossen werden.

Die Fluidsteuerbauteile 21 sind fluiddurchströmt. Beim Ausführungsbeispiel weist der Fluidsteuerkreis 19 ein Schaltventil 26, ein Druckregelventil 27 und ein Proportionalventil 28 als Stellglied auf. Optional kann der Fluidsteuerkreis 19 auch einen Filter 29 aufweisen. Der Filter 29 ist bevorzugt stromabwärts unmittelbar auf den Eingangsanschluss 23 folgend angeordnet.

Der Steuerschaltkreis 20 weist beim Ausführungsbeispiel eine Steuerung 30 auf, die wenigstens ein Steuersignal S für wenigstens ein ansteuerbares Fluidsteuerbauteil 21 bereitstellt. Beispielsgemäß können zumindest das Schaltventil 26 und das Proportionalventil 28 durch jeweils ein Steuersignal S der Steuereinheit 30 angesteuert werden. Der Steuereinheit 30 kann wenigstens ein Eingangssignal E übermittelt werden, beispielsweise kann es sich bei dem wenigstens einen Eingangssignal E um ein Sensorsignal oder Messsignal handeln. Bei dem in Figur 2 veranschaulichten Ausführungsbeispiel sind mehrere Sensoreinheiten 31 vorhanden, die unterschiedliche Eingangssignale E in Form von Sensorsignalen liefern. Beispielsweise kann der Eingangsdruck im Anschluss an den Eingangsanschluss 23 bzw. dem Filter 29 von einem Drucksensor und der Ausgangsdruck am Ausgangsanschluss 24 von einem weiteren Drucksensor erfasst und an die Steuereinheit 30 übermittelt werden.

Außerdem weist die Fluidsteueranordnung 12 beim Ausführungsbeispiel eine Strömungserfassungseinrichtung 32 auf, die einen Volumenstrom oder Massenstrom entlang eines Strömungsmesskanals 33 der Fluidkanalanordnung 22 bzw. den Volumenstrom oder den Massenstrom charakterisierende Signale erfasst. Gemäß dem Hydraulikplan aus Figur 2 weist der Fluidsteuerkreis 19 eine Schaltungsanordnung auf, bei der die Strömungserfassungseinrichtung 32 stromaufwärts von dem Proportionalventil 28 angeordnet ist. Das Proportionalventil 28, das das Stellglied zur Steuerung des Massen- bzw. Volumenstroms zum Instrument 13 darstellt, ist bevorzugt stromaufwärts unmittelbar vor dem Ausgangsanschluss 24 angeordnet. Es ist beispielsgemäß vorgesehen, das Druckregelventil 27 stromaufwärts von der Strömungserfassungseinrichtung 32 anzuordnen, so dass stromaufwärts von der Strömungserfassungseinrichtung 32 ein vorgegebener Fluiddruck herrscht, der eingangsseitig am Strömungsmesskanal 33 anliegt. Stromaufwärts vom Druckregelventil 27 ist das Schaltventil 26 angeordnet, mit dem die Fluidströmung freigegeben oder blockiert werden kann.

Beim Ausführungsbeispiel weist die Strömungserfassungseinrichtung 32 zwei Sensoreinheiten 31 auf, die jeweils einen Differenzdruck zwischen zwei beabstandeten Messstellen im Strömungsmesskanal 33 erfassen und an die Steuereinheit 30 übermitteln. Anhand des Differenzdruckes kann die Steuereinheit 30 einen Volumenstrommesswert oder einen Massenstrommesswert des durch den Strömungsmesskanal 33 strömenden Fluids erfassen. Die Messstellen der beiden Sensoreinheiten 31 sind beispielsgemäß in verschiedenen Kanalabschnitten mit unterschiedlichen Strömungsquerschnitten. Dadurch kann der Gesamtmessbereich zur Ermittlung des Massen- oder Volumenstroms vergrößert werden.

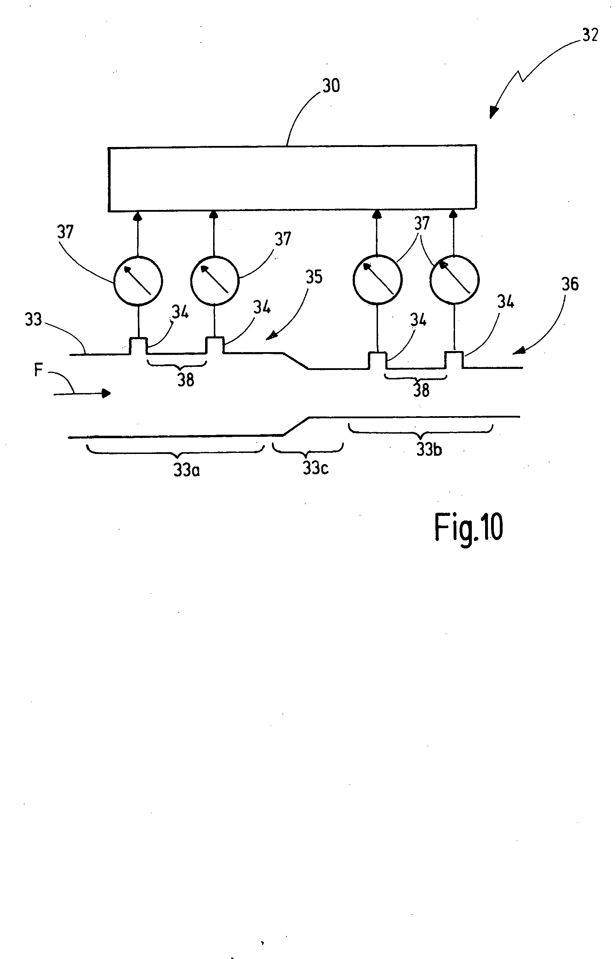

Ein Ausführungsbeispiel für die Strömungserfassungseinrichtung 32 ist schematisch in Figur 10 veranschaulicht. Der Strömungsmesskanal 33 weist einen ersten Kanalabschnitt 33a, einen zweiten Kanalabschnitt 33b und einen dazwischen angeordneten Verbindungskanalabschnitt 33c auf. In Strömungsrichtung F des Fluids sind der erste Kanalabschnitt 33a, der Verbindungskanalabschnitt 33c und der zweite Kanalabschnitt 33b hintereinander angeordnet. Der erste Kanalabschnitt 33a hat einen größeren Strömungsquerschnitt als der zweite Kanalabschnitt 33b. Im Bereich des Verbindungskanalabschnitts 33c verjüngt sich der Strömungsquerschnitt des Strömungsmesskanals 33. Die Querschnittskontur des Strömungsmesskanals 33 ist vorzugsweise im Wesentlichen kreisrund.

In den ersten Kanalabschnitt 33a münden zwei in Strömungsrichtung F mit Abstand angeordnete Druckmesskanäle 34 eines ersten Druckmesskanalpaares 35. In den zweiten Kanalabschnitt 33b des Strömungsmesskanals 33 münden in Strömungsrichtung F mit Abstand zueinander zwei Druckmesskanäle 34 eines zweiten Druckmesskanalpaares 36. Innerhalb eines jeden Kanalabschnitts 33a, 33b ändert sich der Strömungsquerschnitt des Strömungsmesskanals 33 nicht. Durch die Reibung des Fluids beim Strömen entlang des Strömungsmesskanals 33 wird ein Druckverlust zwischen den Druckmesskanälen 34 des jeweiligen Druckmesskanalpaares 35 erzeugt. An jeden Druckmesskanal 34 ist ein Drucksensor 37 angeschlossen, wobei jeder Drucksensor 37 ein Drucksignal erzeugt und an die Steuereinheit 30 weiterleitet, der dem Druck des Fluids an der Stelle entspricht, an der der zugeordnete Druckmesskanal 34 in den Strömungsmesskanal 33 mündet. Die jeweilige Messblende 38 ist bei diesem Ausführungsbeispiel durch die Kanalwand des betreffenden Kanalabschnitts 33a zwischen den Mündungsstellen der Druckmesskanäle 34 eines gemeinsamen Druckmesskanalpaares 35 bzw. 36 gebildet. In der Steuereinheit 30 kann aus zwei Drucksignalen, insbesondere zwei Drucksignalen der Drucksensoren 37, die zu einem gemeinsamen Druckmesskanalpaare 35 bzw. 36 gehören, ein Massenströmungswert oder ein Volumenströmungswert für das Fluid durch den Strömungsmesskanal 33 bestimmt werden.

Es ist auch möglich, an die Druckmesskanäle 34 eines oder jedes Druckmesskanalpaares 35 bzw. 36 einen Differenzdrucksensor anzuschließen.

Die vorstehend beschriebene hydraulische Anordnung der Fluidsteuerbauteile 21 im Fluidsteuerkreis 19 bezüglich der Strömungsrichtung F stellt einen Aspekt der Erfindung dar, der unabhängig von den nachfolgend beschriebenen weiteren Aspekten realisiert werden kann. Durch das Anordnen der Strömungserfassungseinrichtung 32 stromaufwärts von dem Stellglied darstellenden Proportionalventil 28 kann die Messung des Volumenstroms bzw. Massenstroms unabhängig von der Last erfolgen, die beispielsgemäß von dem Instrument 13 gebildet ist. Die Einstellung des Proportionalventils 28 ist in der Steuereinheit 30 bekannt, weil die Einstellung durch ein Steuersignal S der Steuereinheit 30 vorgegeben wird. Die Einstellung kann gegebenenfalls bei der Ermittlung des Volumen- bzw. Massenstromwertes berücksichtigt werden.

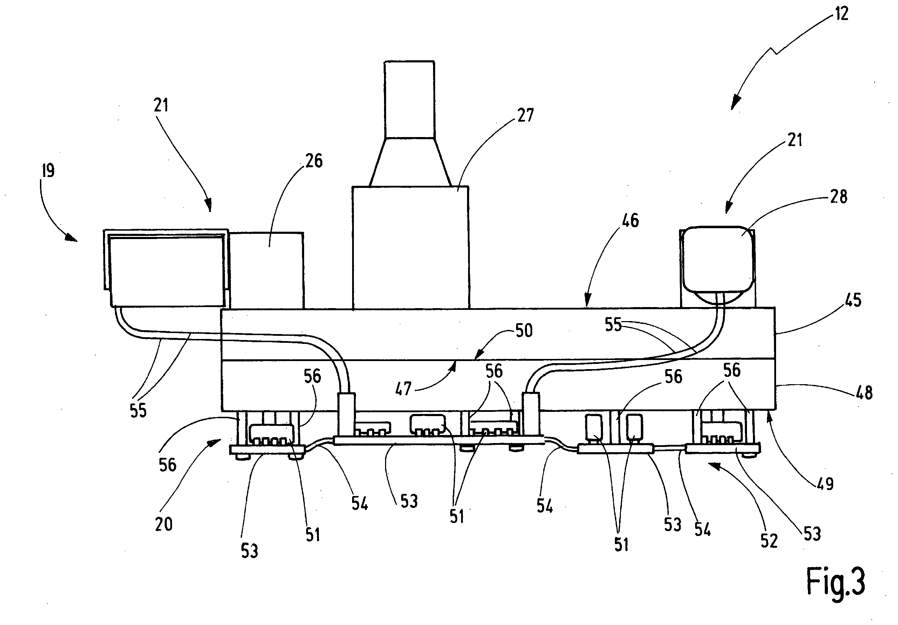

Weitere zusätzliche oder alternative Aspekte der erfindungsgemäßen Fluidsteueranordnung 12 betreffen den kompakten und einfachen Aufbau und/oder die Herstellung und/oder die Montage der Fluidsteueranordnung 12. Diese erfindungsgemäßen Aspekte werden nachfolgend unter Bezugnahme auf die Figuren 3-9 erläutert.

In Figur 3 ist ein Ausführungsbeispiel für den mechanischen Aufbau der Fluidsteueranordnung 12 schematisch veranschaulicht. Die Fluidsteueranordnung 12 hat bei diesem Ausführungsbeispiel ein erstes Trägerteil 45 mit einer ersten Montagefläche 46 und einer ersten Kopplungsfläche 47 sowie ein zweites Trägerteil 48 mit einer zweiten Montagefläche 49 und einer zweiten Kopplungsfläche 50. Die erste Montagefläche 46 ist beim Ausführungsbeispiel dazu eingerichtet, das wenigstens eine Fluidsteuerbauteil 21 des Fluidsteuerkreises 19 zu tragen. Die Fluidsteuerbauteile 21 können insbesondere unmittelbar an der ersten Montagefläche 46 angebracht werden, wie es in Figur 3 veranschaulicht ist. Die erste Montagefläche 46 und die erste Kopplungsfläche 47 sind beispielsgemäß entgegengesetzte Seiten des ersten Trägerteils 45. Analog hierzu sind die zweite Montagefläche 49 und die zweite Kopplungsfläche 50 entgegengesetzte Seiten des zweiten Trägerteils 48.

Vorzugsweise erstreckt sich die erste Montagefläche 46 und/oder die zweite Montagefläche 49 in einer Ebene. Die erste Kopplungsfläche 47 hat beispielsgemäß einen insbesondere zusammenhängenden ebenen Flächenabschnitt 47a der sich in einer Ebene erstreckt, die vorzugsweise parallel zu der Ebene ausgerichtet ist, in der sich die erste Montagefläche 46 erstreckt. Die zweite Kopplungsfläche 50 hat beispielsgemäß einen insbesondere zusammenhängenden ebenen Flächenabschnitt 50a der sich in einer Ebene erstreckt, die vorzugsweise parallel zu der Ebene ausgerichtet ist, in der sich die zweite Montagefläche 49 erstreckt. Die Trägerteile 45, 48 können zum Beispiel plattenförmig oder quaderförmig sein.

Die Kopplungsflächen 47, 50 sind dazu eingerichtet, eine Verbindung zwischen den beiden Trägerteilen 45, 48 herzustellen, wobei die ebenen Flächenabschnitte 47a, 50a der Kopplungsflächen 47, 50 aneinander anliegen können oder einander zugewandt unter Bildung eines Zwischenraums angeordnet sein können.

Die zweite Montagefläche 49 ist beispielsgemäß dazu eingerichtet, wenigstens ein elektrisches und/oder elektronisches Bauteil des Steuerschaltkreises 20 zu tragen. Wie es in Figur 3 veranschaulicht ist, sind mehrere und vorzugsweise sämtliche elektrische und/oder elektronischen Bauteile 51 des Steuerschaltkreises 20 auf einer Leiterplattenanordnung 52 angeordnet, die an der zweiten Montagefläche 49 befestigt ist. Die Leiterplattenanordnung 52 weist mehrere bauteiltragende Leiterplattenabschnitte 53 auf, die mittels flexibler Verbindungsabschnitte 54 elektrisch und mechanisch miteinander verbunden sind. Die flexiblen Verbindungsabschnitte 54 sind integraler Bestandteil der beiden bauteiltragenden Leiterplattenabschnitte 53, die durch den betreffenden flexiblen Verbindungsabschnitt 54 miteinander verbunden werden. Die Leiterplattenanordnung 52 ist daher insgesamt integral ausgebildet und Stecker und Steckverbindungen mit Verbindungskabeln zwischen zwei Leiterplattenabschnitten 53 können entfallen.

Es ist außerdem bevorzugt, wenn die elektrischen und/oder elektronischen Bauteile 51 des Steuerschaltkreises 20 auf der Seite der Leiterplattenanordnung 52 angeordnet sind, die der zweiten Montagefläche 49 zugewandt ist. Vorzugsweise sind auf der der zweiten Montagefläche 49 entgegengesetzten Unterseite der Leiterplattenanordnung 52 keine elektrischen und/oder elektronischen Bauteile 51 vorhanden. Dadurch sind die Bauteile 51 im Zwischenraum zwischen der Leiterplattenanordnung 52 und dem zweiten Trägerteil 48 geschützt angeordnet, was die Handhabung bei der Montage erleichtert.

Wie es in Figur 3 auch zu erkennen ist, können eine oder mehrere elektrische Steuerleitungen 55 vom Steuerschaltkreis 20 zu einem oder mehreren der Fluidsteuerbauteile 21 führen, beispielsgemäß zum Schaltventil 26 und zum Proportionalventil 28. Über diese Steuerleitungen 55 ist eine Ansteuerung des betreffenden Fluidsteuerbauteils 21 mittels des Steuerschaltkreises 20 ermöglicht.

Zu dem wenigstens einen elektrischen und/oder elektronischen Bauteil 51 des Steuerschaltkreises 20 gehört beim Ausführungsbeispiel die Steuereinheit 30 sowie die Drucksensoren 37 der Strömungserfassungseinrichtung 32 und optional zusätzlich die Drucksensoren zur Erfassung des Eingangsdrucks und/oder des Ausgangsdrucks (vergleiche Fig. 2) .

Die Leiterplattenanordnung 52 und beispielsgemäß die bauteiltragenden Leiterplattenabschnitte 53 sind beim Ausführungsbeispiel über ein oder mehrere Montagestifte mit der Montagefläche 49 verbunden. Die Montagestifte 56 sind beispielsgemäß integraler Bestandteil des zweiten Trägerteils 48 und ragen in etwa rechtwinklig von der zweiten Montagefläche 49 weg. Jeder Montagestift 56 kann vorzugsweise einen kreisförmigen Querschnitt aufweisen und an seinem freien Ende ein Innengewinde haben. In den bauteiltragenden Leiterplattenabschnitten können Befestigungslöcher vorhanden sein, die dem Anordnungsmuster der Montagestifte 56 entsprechen. Die bauteiltragenden Leiterplattenabschnitte 53 können an den freien Enden der Montagestifte 56 angelegt und z.B. mittels Schrauben oder anderer geeigneter Befestigungsmittel befestigt werden, wie es schematisch in Figur 3 veranschaulicht ist.

Die erste Kopplungsfläche 47 des ersten Trägerteils 45 weist wenigstens eine erste Fluidkanalaussparung und beim Ausführungsbeispiel mehrere erste Fluidkanalaussparungen 60 auf. Die Fluidkanalaussparungen 60 sind durch rinnenartige Vertiefungen gebildet, die gegenüber dem ebenen Flächenabschnitt 47a der ersten Kopplungsfläche 47 vertieft ausgebildet sind. Im Querschnitt entlang ihrer Erstreckung sind die ersten Fluidkanalaussparungen 60 vorzugsweise halbkreisförmig ausgebildet. Jede erste Fluidkanalaussparung 60 erstreckt sich beim Ausführungsbeispiel geradlinig. Alternativ zum bevorzugten Ausführungsbeispiel könnten eine oder mehrere der ersten Fluidkanalaussparungen 60 auch einen bogenförmigen bzw. gekrümmten Verlauf in ihrer Erstreckungsrichtung entlang der ersten Kopplungsfläche 47 aufweisen.

Im ersten Trägerteil 45 sind außerdem mehrere erste Abzweigkanäle 61 vorhanden, die sich von der ersten Kopplungsfläche 47 bis zur ersten Montagefläche 46 hin erstrecken. Beim Ausführungsbeispiel münden die ersten Abzweigkanäle 61 jeweils in eine der ersten Fluidkanalaussparungen 60. Bevorzugt münden in jede erste Fluidkanalaussparung 60 wenigstens zwei erste Abzweigkanäle 61. Mittels der ersten Abzweigkanäle 61 kann eine Fluidverbindung zwischen einem oder mehreren der Fluidsteuerbauteile 21 und den ersten Fluidkanalaussparungen 60 hergestellt werden.

In der zweiten Kopplungsfläche 50 des zweiten Trägerteils 48 sind beispielsgemäß mehrere zweite Fluidkanalaussparungen 62 vorhanden. Die zweiten Fluidkanalaussparungen 62 können entsprechend den ersten Fluidkanalaussparungen 60 ausgestaltet sein und bilden beim Ausführungsbeispiel rinnenartige Vertiefungen mit einem vorzugsweise halbkreisförmigen Querschnitt in ihrer Erstreckungsrichtung. Die zweiten Fluidkanalaussparungen 62 sind gegenüber dem ebenen Flächenabschnitt 50a der zweiten Kopplungsfläche 50 vertieft ausgestaltet.

Im zweiten Trägerteil 48 können zweite Abzweigkanäle 63 vorhanden sein, die sich vollständig zwischen der zweiten Montagefläche 49 und der zweiten Kopplungsfläche 50 erstrecken und in einen oder mehrere der zweiten Fluidkanalaussparungen 62 münden. Bei dem hier veranschaulichten Ausführungsbeispiel münden alle zweiten Abzweigkanäle 63 in eine einzige zweite Fluidkanalaussparung 62, wobei diese zweiten Abzweigkanäle 63 jeweils einen Druckmesskanal 34 der Strömungserfassungseinrichtung 32 bilden oder Bestandteil des jeweiligen Druckmesskanals 34 sind. Bei dem hier veranschaulichten Ausführungsbeispiel weist jeder Druckmesskanal 34 einen in die zweite Fluidkanalaussparung 62 einmündenden Abschnitt auf, der durch den zweiten Abzweigkanal 63 gebildet ist, wobei sich an den zweiten Abzweigkanal 63 ein weiterer Abschnitt des Druckmesskanals 34 anschließt, der innerhalb eines Stutzens 64 gebildet ist. Der Stutzen 64 erstreckt sich schräg oder rechtwinklig von der zweiten Montagefläche 49 weg zu einem freien Ende 65 hin. An diesem freien Ende 65 ist der dem Druckmesskanal 34 zugeordnete Drucksensor 37 fluidisch mit dem Druckmesskanal 34 gekoppelt.

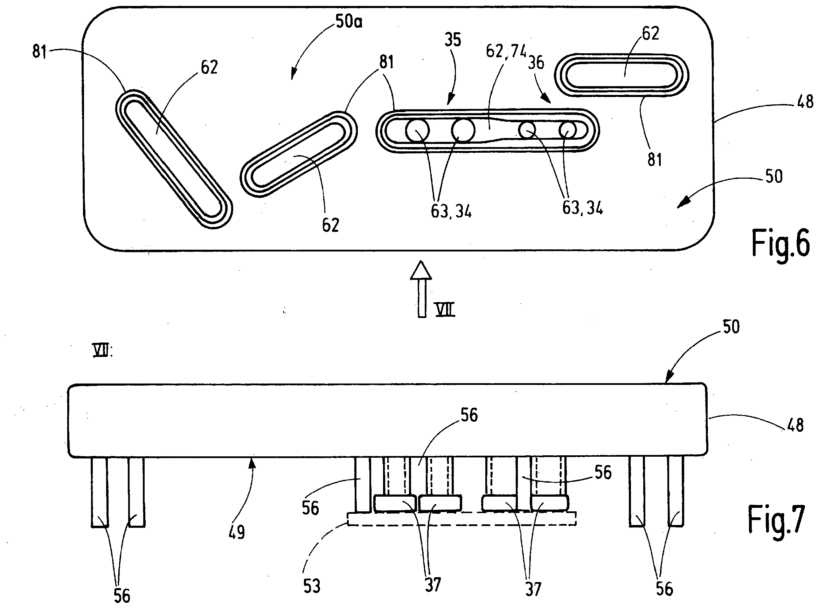

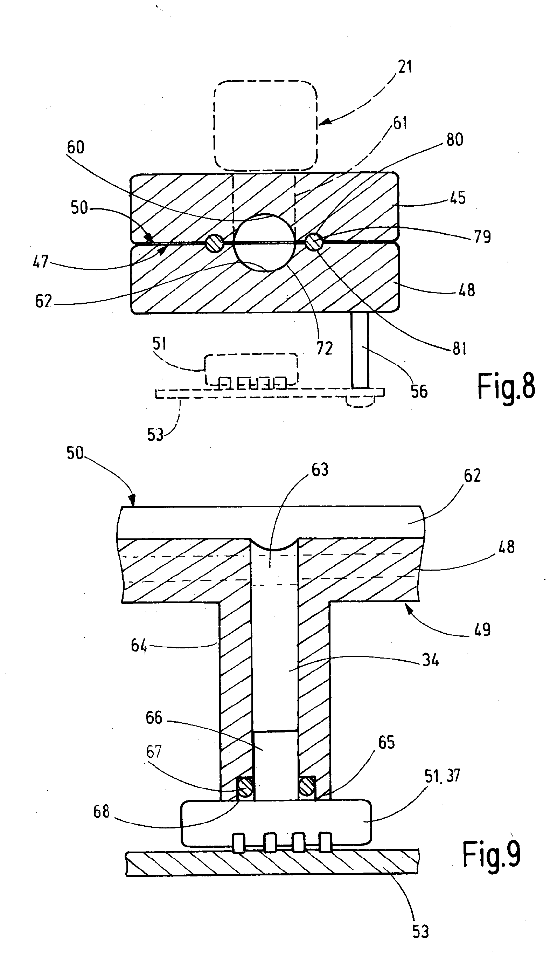

Die Anordnung des Drucksensors im Stutzen 64 ist schematisch in Figur 9 veranschaulicht. Beispielsgemäß weist Drucksensor 37 ein Messelement 66 auf, das in den Druckmesskanal 34 hineinragt und von einem Radialdichtelement 67 ringförmig umschlossen ist. Das Radialdichtelement 67 sitzt in einer Ringaussparung 68 im Bereich des freien Endes 65 des Stutzens 64. Es stützt sich an einer Umfangswand der Ringaussparung 68 an der radialen Außenseite und an dem Messelement 66 an der radialen Innenseite ab, um auf diese Weise eine Radialdichtwirkung zu erzeugen. Wie es in Figur 7 zu erkennen ist, sind sämtliche Drucksensoren 37 der Strömungserfassungseinrichtung 32 an jeweils einem separaten Stutzen 64 angeordnet und werden vorzugsweise von einem gemeinsamen bauteiltragenden Leiterplattenabschnitt 53 getragen.

Die beiden Trägerteile 45, 48 werden mit den einander zugewandten Kopplungsflächen 47, 50 mechanisch miteinander verbunden, wobei der ebenen Flächenabschnitt 47a der ersten Kopplungsfläche 47 am ebenen Flächenabschnitt 50a der zweiten Kopplungsfläche 50 anliegen kann oder unter Bildung eines Spalts oder Zwischenraums angeordnet sein kann. Beispielsgemäß sind die ersten Fluidkanalaussparungen 60 und die zweiten Fluidkanalaussparungen 62 derart gewählt, dass jeweils eine erste Fluidkanalaussparung 60 und eine zweite Fluidkanalaussparung 62 im Bereich der Trennebene bzw. Trennstelle zwischen den beiden Trägerteilen 45, 48 einen Hauptfluidkanal 72 bilden bzw. begrenzen (Figur 8). Da beispielsgemäß vier erste Fluidkanalaussparungen 60 und vier zweite Fluidkanalaussparungen 62 vorhanden sind, ergeben sich vier Hauptfluidkanäle 72.

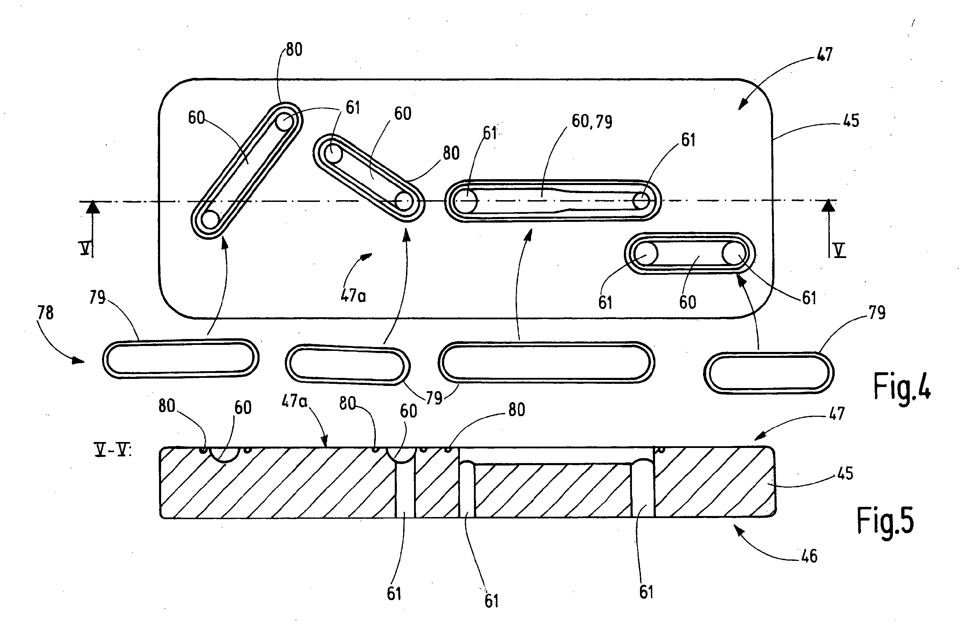

Einer dieser zweiten Hauptfluidkanäle 72 bildet den Strömungsmesskanal 33. Wie es in den Figuren 4 und 6 zu erkennen ist, weist eine der ersten Fluidkanalaussparungen 60 und die zugeordnete zweite Fluidkanalaussparung 62 jeweils einen Abschnitt mit größerem Querschnitt und jeweils einen Abschnitt mit kleinerem Querschnitt auf, die über einen sich verjüngenden, Abschnitt miteinander verbunden sind. Diese Fluidkanalaussparungen können als erste Messkanalaussparung 73 bzw. als zweite Messkanalaussparung 74 bezeichnet werden. In die zweite Messkanalaussparung 74 münden die zwei Druckmesskanäle 34 des ersten Druckmesskanalpaares 35 und die zwei Druckmesskanäle 34 des zweiten Druckmesskanalpaares 36 ein. Zur weiteren Ausgestaltung der Strömungsmesseinrichtung 32 wird auf die vorstehenden Erläuterungen, insbesondere unter Bezugnahme auf die Figuren 2 und 10 verwiesen.

In Abwandlung zum hier dargestellten bevorzugten Ausführungsbeispiel ist es auch möglich, dass eine erste Fluidkanalaussparung 60 mit dem ebenen Flächenabschnitt 50a der zweiten Kopplungsfläche 50 einen Hauptfluidkanal 72 begrenzt und/oder dass eine zweite Fluidkanalaussparung 62 mit dem ebenen Flächenabschnitt 47a der ersten Kopplungsfläche 47 einen Hauptfluidkanal 72 begrenzt. Es müssen daher nicht notwendigerweise übereinstimmende erste und zweite Fluidkanalaussparungen 60 und 62 zur Bildung des Hauptfluidkanals 72 vorhanden sein.

Zur Abdichtung der beiden Trägerteile 45, 48 im Bereich der Kopplungsflächen 47, 50 ist eine Dichtungsanordnung 78 vorhanden, die beispielhaft in Figur 4 veranschaulicht ist. Beim Ausführungsbeispiel hat die Dichtungsanordnung 78 mehrere separate Ringdichtungen 79. Jede Ringdichtung 79 wird beim Ausführungsbeispiel in eine erste Ringnut 80 an der ersten Kopplungsfläche 47 des ersten Trägerteils 45 eingelegt. Die erste Ringnut 80 umschließt jeweils eine erste Fluidkanalaussparung 60 vollständig. Die erste Ringnut 80 ist derat dimensioniert, dass die eingelegte Ringdichtung 79 aus der ersten Ringnut 80 herausragt und über den ebenen Flächenabschnitt 47a der ersten Kopplungsfläche 47 vorsteht.

Beim Ausführungsbeispiel sind auch in der zweiten Kopplungsfläche 50 zweite Ringnuten 81 vorhanden, die jeweils eine zweite Fluidkanalaussparung 62 vollständig umschließen. Die zweiten Ringnuten 81 können entsprechend der ersten Ringnuten 80 ausgebildet sein. Wenn das erste Trägerteil 45 und das zweite Trägerteil 48 mit ihren einander zugewandten Kopplungsflächen 47, 50 miteinander verbunden, sitzt jede Ringdichtung 79 in einer ersten Ringnut 80 und in einer zweiten Ringnut 81, wie es schematisch in Figur 8 veranschaulicht ist. Dadurch ist eine sehr gute Positionierung der Ringdichtung 79 und eine entsprechende Dichtwirkung sichergestellt.

In Abwandlung zum bevorzugten Ausführungsbeispiel könnte auch lediglich in einem der Trägerteile 45, 48 die wenigstens eine Ringnut 80 oder 81 vorhanden sein, um einen nach dem Zusammenfügen der Trägerteile 45, 48 gebildeten Hauptfluidkanal 72 abzudichten. In einer weiteren Abwandlung könnte ein plattenförmiges Dichtelement zwischen den beiden Trägerteilen 45, 48 angeordnet werden, das im Bereich der ersten Fluidkanalaussparungen 60 und der zweiten Fluidkanalaussparungen 62 entsprechende Aussparungen bzw. Durchbrechungen aufweist und um die ersten Fluidkanalaussparungen 60 und der zweiten Fluidkanalaussparungen 62 herum an den ebenen Flächenabschnitten 47a, 50a der Kopplungsflächen 47, 50 anliegt.

Bei einem anderen Ausführungsbeispiel kann die Dichtungsanordnung 78 auch durch eine stoffschlüssige Verbindung oder Haftvermittlungsverbindung mit einem der Trägerteile verbunden sein. Beispielsweise kann die Dichtungsanordnung 78 an eine der Kopplungsflächen 47, 50 angeklebt werden oder beim Herstellen der Trägerteile 45, 48 durch ein Zweikomponenten-Spritzgießverfahren an dem Trägerteil 45, 48 angebracht werden.

Bei einem bevorzugten Ausführungsbeispiel sind die Trägerteile 45, 48 zumindest im Bereich ihrer Kopplungsflächen 47, 50 oder alternativ vollständig aus einem transparenten Material hergestellt. Dadurch kann im zusammengefügten, montierten Zustand eine Sichtprüfung erfolgen, ob die Ringdichtungen 79 korrekt in den jeweils zugeordneten Ringnuten 80 bzw. 81 liegen und die Dichtwirkung sichergestellt ist.

Ein weiterer, unabhängiger Aspekt der erfindungsgemäßen Fluidsteueranordnung 12 betrifft das Herstellen der Trägerteile 45, 48. Vorzugsweise sind die Trägerteile 45, 48 als Spritzgussteile ausgebildet. Dabei wird die Formschließrichtung vorzugsweise derart gewählt, dass die Formschließrichtung mit der Erstreckungsrichtung der Abzweigkanäle 61, 63 übereinstimmt. Die Formschließrichtung ist beim Spritzgießen die Richtung, in der die beiden Spritzgussformteile zum Schließen der Spritzgussform bzw. zum Öffnen der Spritzgussform relativ zueinander bewegt werden.

Jeder Abzweigkanal 61, 63 eines Trägerteils 45, 48 ist in einer Erstreckungsrichtung von einem Ende zum jeweils anderen Ende hinterschneidungsfrei ausgebildet. In dieser Erstreckungsrichtung kann der Abzweigkanal 61, 63 konisch verjüngt ausgebildet sein. Jeder erste Abzweigkanal 61 erstreckt sich entweder hinterschneidungsfrei ausgehend von der ersten Montagefläche 46 zur ersten Kopplungsfläche 47 oder umgekehrt von der ersten Kopplungsfläche 47 zur ersten Montagefläche 46. Analog hierzu erstreckt sich jeder zweite Abzweigkanal 63 hinterschneidungsfrei von der ersten Kopplungsfläche 50 zur ersten Montagefläche 49 oder bis zum freien Ende des Stutzens 64 oder umgekehrt vom freien Ende des Stutzens 64 oder von der zweiten Montagefläche 49 zur zweiten Kopplungsfläche 50. Durch diese hinterschneidungsfreie Ausgestaltung der Abzweigkanäle 61, 63 kann das Trägerteil 45, 48 sehr einfach als Spritzgussteil ausgebildet werden.

Die Erfindung betrifft eine Fluidsteueranordnung 12 für ein medizinisches Gerät 10, insbesondere ein medizinisches Gerät zur Argonplasma-Koagulation. Die Fluidsteueranordnung 12 hat einen Fluidsteuerkreis 19 mit wenigstens einem fluiddurchströmten Fluidsteuerbauteil 21. Zur Steuerung des wenigstens einen Fluidsteuerbauteils 21 ist ein Steuerschaltkreis 20 mit wenigstens einem elektrischen und/oder elektronischen Bauteil 51 vorhanden. Ein erstes Trägerteil 45 hat eine erste Kopplungsfläche 47 und ein zweites Trägerteil 48 hat eine zweite Kopplungsfläche 50. In der ersten Kopplungsfläche 47 ist wenigstens eine erste Fluidkanalaussparung 60 und/oder in der zweiten Kopplungsfläche 47 ist wenigstens eine zweite Fluidkanalaussparung 62 vorhanden. Beim Verbinden der Trägerteile 45, 48 miteinander zugewandten Kopplungsflächen 47, 50 wird dadurch im Bereich der Trennstelle wenigstens ein Hauptfluidkanal 72 gebildet. Jeder Hauptfluidkanal 72 ist somit teilweise durch das erste Trägerteil 45 und teilweise durch das zweite Trägerteil 48 begrenzt. Am ersten Trägerteil 45 kann eine erste Montagefläche 46 für das wenigstens eine Fluidsteuerbauteil 21 vorhanden sein. Am zweiten Trägerteil 48 kann eine zweite Montagefläche 49 für das wenigstens eine elektrische und/oder elektronische Bauteil 51 des Steuerschaltkreises 20 vorhanden sein. Zusätzlich oder alternativ zu dem Montieren von Bauteilen an den Montageflächen 46, 49 können die Trägerteile 45, 48 als Spritzgussteile ausgebildet sein. Ein weiterer unabhängiger Aspekt betrifft die Hydraulikschaltung im Fluidsteuerkreis 19, wobei eine Strömungserfassungseinrichtung 32 stromaufwärts von einem Stellglied angeordnet ist, über das gesteuert durch den Steuerschaltkreis 20 die Fluidströmung aus einem Ausgangsanschluss 24 der Fluidsteueranordnung 12 gesteuert oder geregelt werden kann.

### Bezugszeichenliste:

- 10: medizinisches Gerät
- 11: Fluidquelle
- 12: Fluidsteueranordnung
- 13: Instrument
- 14: Hochspannungsquelle
- 15: Elektrode

- 19: Fluidsteuerkreis
- 20: Steuerschaltkreis
- 21: Fluidsteuerbauteil
- 22: Fluidkanalanordnung
- 23: Eingangsanschluss
- 24: Ausgangsanschluss

- 26: Schaltventil
- 27: Druckregelventil
- 28: Proportionalventil
- 29: Filter
- 30: Steuereinheit
- 31: Sensoreinheiten
- 32: Strömungserfassungseinrichtung
- 33: Strömungsmesskanal
- 33a: erster Kanalabschnitt
- 33b: zweiter Kanalabschnitt
- 33c: Verbindungskanalabschnitt
- 34: Druckmesskanal
- 35: erstes Druckmesskanalpaar
- 36: zweites Druckmesskanalpaar
- 37: Drucksensor
- 38: Messblende
- 45: erstes Trägerteil
- 46: erste Montagefläche
- 47: erste Kopplungsfläche
- 47a: ebener Flächenabschnitt der ersten Kopplungsfläche
- 48: zweites Trägerteil
- 49: zweite Montagefläche
- 50: zweite Kopplungsfläche
- 50a: ebener Flächenabschnitt der zweiten Kopplungsfläche
- 51: elektrisches und/oder elektronisches Bauteil
- 52: Leiterplattenanordnung
- 53: bauteiltragender Leiterplattenabschnitt
- 54: flexibler Verbindungsabschnitt
- 55: Steuerleitung
- 56: Montagestift

- 60: erste Fluidkanalaussparung
- 61: erster Abzweigkanal
- 62: zweite Fluidkanalaussparung
- 63: zweiter Abzweigkanal
- 64: Stutzen
- 65: freies Ende des Stutzens
- 66: Messelement
- 67: Radialdichtelement
- 68: Ringaussparung

- 72: Hauptfluidkanal
- 73: erste Messkanalaussparung
- 74: zweite Messkanalaussparung

- 78: Dichtungsanordnung
- 79: Ringdichtung
- 80: erste Ringnut
- 81: zweite Ringnut

- E: Eingangssignal
- F: Strömungsrichtung
- S: Steuersignal

## Patentansprüche

1. Fluidsteueranordnung (12) für ein medizinisches Gerät (10),
mit einem Fluidsteuerkreis (19), der eine Fluidkanalanordnung (22) und wenigstens ein Fluidsteuerbauteil (21) aufweist, die zwischen einem Eingangsanschluss (23) und einem Ausgangsanschluss (24) angeordnet sind,
mit einem wenigstens ein elektrisches und/oder elektronisches Bauteil (51) aufweisenden Steuerschaltkreis (20), der dazu eingerichtet ist, das wenigstens eine Fluidsteuerbauteil (21) des Fluidsteuerkreises (19) zu steuern,
mit einem ersten Trägerteil (45), das eine erste Montagefläche (46) und eine erste Kopplungsfläche (47) aufweist und mit einem zweiten Trägerteil (48), das eine zweite Montagefläche (49) und eine zweite Kopplungsfläche (50) aufweist,
wobei das wenigstens eine Fluidsteuerbauteil (21) des Fluidsteuerkreises (19) und das wenigstens eine elektrische und/oder elektronische Bauteil (51) des Steuerschaltkreises (20) an den Montageflächen (46, 49) der Trägerteile (45, 48) angeordnet sind,
**dadurch gekennzeichnet, dass** wenigstens eine erste Fluidkanalaussparung (60) in der ersten Kopplungsfläche (47) und/oder wenigstens eine zweite Fluidkanalaussparung (62) in der zweiten Kopplungsfläche (50) vorhanden ist,
dass die beiden Trägerteile (45, 48) derart an Kopplungsflächen (47, 50) miteinander verbunden sind, dass die wenigstens eine erste Fluidkanalaussparung (60) und/oder die wenigstens eine zweite Fluidkanalaussparung (62) wenigstens einen Hauptfluidkanal (72) der Fluidkanalanordnung (22) begrenzen,
und dass zwischen der ersten Kopplungsfläche (47) und der zweiten Kopplungsfläche (50) eine Dichtungsanordnung (78) zur Abdichtung des wenigstens einen Hauptfluidkanals angeordnet ist.

2. Fluidsteueranordnung nach Anspruch 1,
**dadurch gekennzeichnet, dass** sowohl wenigstens eine erste Fluidkanalaussparung (60) als auch wenigstens eine zweite Fluidkanalaussparung (62) vorhanden sind, wobei jeweils eine erste Fluidkanalaussparung (60) und eine zugeordnete zweite Fluidkanalaussparung (62) einen Hauptfluidkanal (72) der Fluidkanalanordnung (22) bilden.

3. Fluidsteueranordnung nach Anspruch 2,
**dadurch gekennzeichnet, dass** jede erste Fluidkanalaussparung (60) als auch jede zweite Fluidkanalaussparung (62) jeweils einen halbkreisförmigen Querschnitt aufweist.

4. Fluidsteueranordnung nach einem der vorhergehenden Ansprüche,
**dadurch gekennzeichnet, dass** das erste Trägerteil (45) und das zweite Trägerteil (48) durch jeweils ein Spritzgussteil gebildet sind.

5. Fluidsteueranordnung nach einem der vorhergehenden Ansprüche,
**dadurch gekennzeichnet, dass** in dem ersten Trägerteil (45) wenigstens ein fluidischer erster Abzweigkanal (61) vorhanden ist, der sich zwischen der ersten Montagefläche (46) und der ersten Kopplungsfläche (47) erstreckt und zumindest in einer Erstreckungsrichtung von der ersten Montagefläche (46) zur ersten Kopplungsfläche (47) oder von der ersten Kopplungsfläche (47) zur ersten Montagefläche (46) hinterschneidungsfrei ist.

6. Fluidsteueranordnung nach einem der vorhergehenden Ansprüche,
**dadurch gekennzeichnet, dass** in dem zweiten Trägerteil (48) wenigstens ein fluidischer zweiter Abzweigkanal (63) vorhanden ist, der sich zwischen der zweiten Montagefläche (49) und der zweiten Kopplungsfläche (50) erstreckt und zumindest in einer Erstreckungsrichtung von der zweiten Montagefläche (49) zur zweiten Kopplungsfläche (50) oder von der zweiten Kopplungsfläche (50) zur zweiten Montagefläche (49) hinterschneidungsfrei sind.

7. Fluidsteueranordnung nach einem der vorhergehenden Ansprüche,
**dadurch gekennzeichnet, dass** eine oder mehrere erste Fluidkanalaussparungen (60) und/oder eine oder mehrere zweite Fluidkanalaussparungen (62) mehrere Hauptfluidkanäle (72) bilden, die in den Kopplungsflächen (47, 50) voneinander getrennt sind.

8. Fluidsteueranordnung nach Anspruch 7,
**dadurch gekennzeichnet, dass** die Dichtungsanordnung (78) mehrere Ringdichtungen (79) aufweist, wobei jede Ringdichtung (79) einen Hauptfluidkanal (72) der Fluidkanalanordnung (22) umgibt.

9. Fluidsteueranordnung nach einem der vorhergehenden Ansprüche,
**dadurch gekennzeichnet, dass** jedes Fluidsteuerbauteil (21) an der ersten Montagefläche (46) angeordnet sind.

10. Fluidsteueranordnung nach einem der vorhergehenden Ansprüche,
**dadurch gekennzeichnet, dass** jedes elektrische und/oder elektronische Bauteil (51) an der zweiten Montagefläche (49) angeordnet ist.

11. Fluidsteueranordnung nach einem der vorhergehenden Ansprüche,
**dadurch gekennzeichnet, dass** ein Hauptfluidkanal (72) der Fluidkanalanordnung (22) einen Strömungsmesskanal (33) bildet, dass in wenigstens einem der Trägerteile (45, 48) wenigstens ein Druckmesskanalpaar (35, 36) mit zwei Druckmesskanälen (34) vorhanden ist, die in Strömungsrichtung (F) mit Abstand zueinander in den Strömungsmesskanal (33) münden.

12. Fluidsteueranordnung nach Anspruch 11,
**dadurch gekennzeichnet, dass** an jeden der Druckmesskanäle (34) ein separater Drucksensor (37) angeschlossen ist oder dass an die Druckmesskanäle (34) eines Druckmesskanalpaares (35, 36) ein Differenzdrucksensor angeschlossen ist.

13. Fluidsteueranordnung nach Anspruch 11 oder 12,
**dadurch gekennzeichnet, dass** ein erstes Druckmesskanalpaar (35) vorhanden ist, dessen Druckmesskanäle (34) in einen einen ersten Strömungsquerschnitt aufweisenden ersten Kanalabschnitt (33a) des Strömungsmesskanals (33) münden, und dass ein zweites Druckmesskanalpaar (36) vorhanden ist, dessen Druckmesskanäle (34) in einen einen zweiten Strömungsquerschnitt aufweisenden zweiten Kanalabschnitt (33b) des Strömungsmesskanals (33) münden, wobei der erste und der zweite Strömungsquerschnitt verschieden groß sind.

14. Fluidsteueranordnung nach Anspruch 13,
**dadurch gekennzeichnet, dass** zumindest zwei der von den Drucksensoren (37) gemessenen Druckwerte an eine Steuereinheit (30 oder eine Auswerteeinheit des Steuerschaltkreises (20) übermittelt werden, die dazu eingerichtet ist, daraus einen Massen- oder Volumenströmungswert der Fluidströmung durch den Strömungsmesskanal (33) zu ermitteln.

## Claims

1. Fluid control arrangement (12) for a medical device (10)
having a fluid control circuit (19) comprising a fluid channel arrangement (22) and at least one fluid control component (21) that are arranged between an inlet connection (23) and an outlet connection (24),
having a control circuitry (20) comprising at least one electric and/or electronic component (51), the control circuitry (20) being configured to control the at least one fluid control component (21) of the fluid control circuit (19),
having a first carrier part (45) comprising a first mounting surface (46) and a first coupling surface (47) and having a second carrier part (48) comprising a second mounting surface (49) and a second coupling surface (50),
wherein the at least one fluid control component (21) of the fluid control circuit (19) and the at least one electric and/or electronic component (51) of the control circuitry (20) are arranged at the mounting surfaces (46, 49) of the carrier parts (45, 48),
**characterized in that** at least one first fluid channel cavity (60) is provided in the first coupling surface (47) and/or at least one second fluid channel cavity (62) is provided in the second coupling surface (50),
that the two carrier parts (45, 48) are connected with each other at the coupling surfaces (47, 50) so that the at least one first fluid channel cavity (60) and/or the at least one second fluid channel cavity (62) limit at least one main fluid channel (72) of the fluid channel arrangement (22),
and that a seal arrangement (78) for sealing the at least one main fluid channel (72) is arranged between the first coupling surface (47) and the second coupling surface (50) .

2. Fluid control arrangement according to claim 1, **characterized in that** at least one first fluid channel cavity (60), as well as at least one second fluid channel cavity (62) are provided, wherein each of the first fluid channel cavities (60) and one associated second fluid channel cavity (62) form one main fluid channel (72) of the fluid channel arrangement (22) .

3. Fluid control arrangement according to claim 2, **characterized in that** each first fluid channel cavity (60), as well as each second fluid channel cavity (62) has a semicircle cross-section.

4. Fluid control arrangement according to any of the preceding claims, **characterized in that** the first carrier part (45) and the second carrier part (48) are formed by an injection mold part respectively.

5. Fluid control arrangement according to any of the preceding claims, **characterized in that** in the first carrier part (45) at least one fluidic first branch channel (61) is present that extends between the first mounting surface (46) and the first coupling surface (47) and that is free of undercuts in at least one extension direction from the first mounting surface (46) to the first coupling surface (47) or from the first coupling surface (47) to the first mounting surface (46).

6. Fluid control arrangement according to any of the preceding claims, **characterized in that** in the second carrier part (48) at least one fluidic second branch channel (63) is present that extends between the second mounting surface (49) and the second coupling surface (50) and that is free of undercuts in at least one extension direction from the second mounting surface (49) to the second coupling surface (50) or from the second coupling surface (50) to the second mounting surface (49).

7. Fluid control arrangement according to any of the preceding claims, **characterized in that** one or more first fluid channel cavities (60) and/or one or more second fluid channel cavities (62) form multiple main fluid channels (72) that are separated from each other in the coupling surfaces (47, 50).

8. Fluid control arrangement according to claim 7, **characterized in that** the seal arrangement (78) comprises multiple ring seals (79), wherein each ring seal (79) surrounds a main fluid channel (72) of the fluid channel arrangement (22).

9. Fluid control arrangement according to any of the preceding claims, **characterized in that** each fluid control component (21) is arranged on the first mounting surface (46).

10. Fluid control arrangement according to any of the preceding claims, **characterized in that** each electric and/or electronic component (51) is arranged on the second mounting surface (49).

11. Fluid control arrangement according to any of the preceding claims, **characterized in that** a main fluid channel (72) of the fluid channel arrangement (22) forms a flow measurement channel (33), that in at least one of the carrier parts (45, 48) at least one pressure measurement channel pair (35, 36) with two pressure measurement channels (34) is provided, which open into the flow measurement channel (33) with distance to each other in flow direction (F).

12. Fluid control arrangement according to claim 11, **characterized in that** a separate pressure sensor (37) is connected to each of the pressure measurement channels (34) or that a difference pressure sensor is connected to the pressure measurement channels (34) of one pressure measurement channel pair (35, 36).

13. Fluid control arrangement according to claim 11 or 12, **characterized in that** a first pressure measurement channel pair (35) is provided, the pressure measurement channels (34) of which open into a first channel section (33a) of the flow measurement channel (33) having a first flow cross-section and that a second pressure measurement channel pair (36) is provided, the pressure measurement channels (34) of which open into a second channel section (33b) of the flow measurement channel (33) having a second flow cross-section, wherein the first and the second flow cross-sections have different areas.

14. Fluid control arrangement according to claim 13, **characterized in that** at least two pressure values measured by the pressure sensors (37) are transmitted to a control unit (30) or an evaluation unit of the control circuitry (20), which is configured to determine a mass or volume flow value of the fluid flow through the flow measurement channel (33) from the at least two pressure values.

## Revendications

1. Dispositif de commande de fluide (12) destiné à un appareil médical (10),
comprenant un circuit de commande de fluide (19) qui présente un système de canaux de fluide (22) et au moins un composant de commande de fluide (21) qui sont disposés entre un raccord d'entrée (23) et un raccord de sortie (24),
comprenant un circuit de commande (20) présentant au moins un composant électrique et/ou électronique (51) et conçu pour commander le composant de commande de fluide (21), au nombre d'au moins un, du circuit de commande de fluide (19),
comprenant un premier élément de support (45), qui présente une première surface de montage (46) et une première surface de couplage (47), et comprenant un deuxième élément de support (48) qui présente une deuxième surface de montage (49) et une deuxième surface de couplage (50),
le composant de commande de fluide (21), au nombre d'au moins un, du circuit de commande de fluide (19) et le composant électrique et/ou électronique (51), au nombre d'au moins un, du circuit de commande (20) étant disposés sur les surfaces de montage (46, 49) des éléments de support (45, 48),
**caractérisé en ce qu'**il est prévu au moins un premier évidement de canal de fluide (60) dans la première surface de couplage (47) et/ou au moins un deuxième évidement de canal de fluide (62) dans la deuxième surface de couplage (50),
**en ce que** les deux éléments de support (45, 48) sont reliés l'un à l'autre par des surfaces de couplage (47, 50) de manière telle que le premier évidement de canal de fluide (60), au nombre d'au moins un, et/ou le deuxième évidement de canal de fluide (62), au nombre d'au moins un, délimitent au moins un canal de fluide principal (72) du système de canaux de fluide (22),
et **en ce qu'**entre la première surface de couplage (47) et la deuxième surface de couplage (50), il est prévu un dispositif d'étanchéité (78) destiné à rendre étanche le canal de fluide principal, au nombre d'au moins un.

2. Dispositif de commande de fluide selon la revendication 1, **caractérisé en ce que**, aussi bien au moins un premier évidement de canal de fluide (60) qu'au moins un deuxième évidement de canal de fluide (62) sont prévus, sachant que respectivement un premier évidement de canal de fluide (60) et un deuxième évidement de canal de fluide (62) associé forment un canal de fluide principal (72) du système de canaux de fluide (22).

3. Dispositif de commande de fluide selon la revendication 2, **caractérisé en ce que** chaque premier évidement de canal de fluide (60) ainsi que chaque deuxième évidement de canal de fluide (62) présente respectivement une section transversale en forme de demi-cercle.

4. Dispositif de commande de fluide selon l'une des revendications précédentes, **caractérisé en ce que** le premier élément de support (45) et le deuxième élément de support (48) sont constitués respectivement d'un élément moulé par injection.

5. Dispositif de commande de fluide selon l'une des revendications précédentes, **caractérisé en ce que** dans le premier élément de support (45), il est prévu au moins un premier canal de branchement fluidique (61) qui s'étend entre la première surface de montage (46) et la première surface de couplage (47) et est exempt de contre-dépouilles au moins dans une première dimension d'extension depuis la première surface de montage (46) vers la première surface de couplage (47) ou depuis la première surface de couplage (47) vers la première surface de montage (46).

6. Dispositif de commande de fluide selon l'une des revendications précédentes, **caractérisé en ce que** dans le deuxième élément de support (48), il est prévu au moins un deuxième canal de branchement fluidique (63) qui s'étend entre la deuxième surface de montage (49) et la deuxième surface de couplage (50) et est exempt de contre-dépouilles au moins dans une première dimension d'extension depuis la deuxième surface de montage (49) vers la deuxième surface de couplage (50) ou depuis la deuxième surface de couplage (50) vers la deuxième surface de montage (49).

7. Dispositif de commande de fluide selon l'une des revendications précédentes, **caractérisé en ce qu'**un ou plusieurs premiers évidements de canal de fluide (60) et/ou un ou plusieurs deuxièmes évidements de canal de fluide (62) forment plusieurs canaux de fluide principaux (72) qui sont séparés les uns des autres dans les surfaces de couplage (47, 50).

8. Dispositif de commande de fluide selon la revendication 7, **caractérisé en ce que** le dispositif d'étanchéité (78) présente plusieurs joints toriques (79), chaque joint torique (79) entourant un canal de fluide principal (72) du dispositif de canaux de fluide (22).

9. Dispositif de commande de fluide selon l'une des revendications précédentes, **caractérisé en ce que** chaque composant de commande de fluide (21) est disposé sur la première surface de montage (46).

10. Dispositif de commande de fluide selon l'une des revendications précédentes, **caractérisé en ce que** chaque composant électrique et/ou électronique (51) est disposé sur la deuxième surface de montage (49).

11. Dispositif de commande de fluide selon l'une des revendications précédentes, **caractérisé en ce qu'**un canal de fluide principal (72) du dispositif de canal de fluide (22) forme un canal de mesure de débit (33), **en ce que** dans au moins un des éléments de support (45, 48), il est prévu au moins une paire de canaux de mesure de pression (35, 36) comportant deux canaux de mesure de pression (34) qui, dans le sens d'écoulement (F), débouchent dans le canal de mesure de débit (33), en étant espacés l'un de l'autre.

12. Dispositif de commande de fluide selon la revendication 11, **caractérisé en ce qu'**un capteur de pression (37) distinct est raccordé à chacun des canaux de mesure de pression (34), ou **en ce qu'**un capteur de pression différentielle est raccordé aux canaux de mesure de pression (34) d'une paire de canaux de mesure de pression (35, 36).

13. Dispositif de commande de fluide selon la revendication 11 ou 12, **caractérisé en ce qu'**il est prévu une première paire de canaux de mesure de pression (35), dont les canaux de mesure de pression (34) débouchent dans une première portion de canal (33a) du canal de mesure de débit (33), présentant une première section d'écoulement, et **en ce qu'**il est prévu une deuxième paire de canaux de mesure de pression (36), dont les canaux de mesure de pression (34) débouchent dans une deuxième portion de canal (33b) du canal de mesure de débit (33) présentant une deuxième section d'écoulement, la première et la deuxième section d'écoulement étant différentes l'une de l'autre.

14. Dispositif de commande de fluide selon la revendication 13, **caractérisé en ce qu'**au moins deux des valeurs de pression mesurées par les capteurs de pression (37) sont transmises à une unité de commande (30) ou à une unité d'évaluation du circuit de commande (20), qui est conçue pour déterminer, à partir de ces valeurs, une valeur de débit massique ou volumétrique de l'écoulement de fluide traversant le canal de mesure de débit (33).
